# EUROPEAN PATENT APPLICATION

(11) **EP 3 805 358 A1**
(43) Date of publication of application: **14.04.2021**
(21) Application number: 19306314.6
(22) Date of filing: 08.10.2019
(51) Int. Cl.: C12M 1/16, A23L 7/104, C12M 1/00, C12M 1/12, C12M 1/36

(54) **PROCESS AND PRODUCTION SYSTEM FOR LARGE SCALE SOLID-STATE FERMENTATION**

(71) Applicant: Green Spot Technologies SAS, 31520 Ramonville-Saint-Agne (FR)
(72) Inventor: GRANUCCI, Ninna, 31400 TOULOUSE (FR); GRANATO VILLAS BOAS, Silas, 31500 TOULOUSE (FR)
(74) Representative: Santarelli

(57) **Abstract**

The invention relates to a process for large scale solid-state fermentation. The process comprises providing a substrate to be cultured (S1) made of plant material and/or animal material, filling vessels (S2) with the substrate using an automated filling system, sterilizing (S4) the vessels, inoculating (S5) the substrate with a microbial inoculant adapted to cause fermentation of the cultured substrate, storing (S6) the vessels in a closed state in controlled climate conditions for solid state fermentation of the cultured substrate, and harvesting (S7) the content of the vessels. Each vessel has a smallest dimension less than or equal to 50 cm. This process is particularly adapted, with additional steps, for the production of fermented flour. In this process, upscaling is obtained by providing a high number of small bioreactors and by automation, instead of increasing the size of the reactors as generally done in the field of bioprocessing. The invention also relates to a corresponding production process.

## Description

The present invention relates to a process for large scale solid-state fermentation using microorganisms.

Solid state fermentation (SSF) refers to the microbial fermentation that takes place in the absence or near absence of free water. During Solid State Fermentation, the growth of selected microorganism(s) occurs on solid materials. Solid state fermentation thus takes place in an environment close to the natural growth environment of selected microorganisms, such as filamentous organisms.

This technology has been known for a long time and commonly used in some parts of the world to produce traditional fermented foods or mould-ripened cheese. Traditional fermented food products comprise for example fermented soybean (tempe, soy sauce, annatto, miso, etc.) and fermented rice (tapai, koji, red fermented rice, brewing of the Japanese rice wine (sake)). But Solid-State Fermentation has many applications, such as the production of enzymes, chemicals, biogas, bioethanol and pharmaceuticals. More generally, Solid-State Fermentation may be used for the production of important biomolecules and products for many industries, including food, pharmaceutical, textile, biochemical and bioenergy. In the food industry, Solid-State Fermentation may be used for the production of fermented flours having high nutritional interest.

The main counterpart for "Solid-State Fermentation" is "submerged or liquid fermentation", a process in which microorganisms grow in liquid medium, with high content of free water. Biological processes carried out in submerged or liquid fermentation have notable advantages regarding instrumentation and control (monitoring of pH, dissolved oxygen, temperature, concentration of water-soluble molecules), separation of biomass after the fermentation, mixing, aeration and scaling up. Although scale-up methods for submerged or liquid fermentation are well developed, these methods cannot be applied directly to Solid-State Fermentation due to the differences in the physical structures of the systems used for those respective types of fermentation.

Solid-State Fermentation has however been identified in the scientific literature as a promising technology for bioconversion and biodegradation processes. Solid-Sate Fermentation is a growing technology in the bioprocessing field due to the merits of being environmental friendly, water saving, and energy saving.

While this technology is used at small scales, such as in laboratories or for small or artisanal productions, developing a large scale process for large scale solid-state fermentation using microorganisms remains a challenge that is not fully, satisfactorily, resolved in the state of the art. By large-scale production, it is meant industrial production.

High-solid loading in Solid-State Fermentation processes results in a series of problems such as high viscosity, high stirring energy consumption, and low conversion, which increase with the increased scale.

Difficulties in scale-up of Solid-State Fermentation are in particular related to the heat generation during Solid-State Fermentation, oxygen availability and heterogeneity of the system.

Heat build-up is indeed the typical effect in solid-state fermentation. Because of poor heat conductivity and of accumulation of metabolic heat in the fermenting material (i.e. a cultured substrate) combined with substrate shrinkage and decreased porosity, gas convection is seriously hindered. This effect is undesirable especially in some biotechnological processes of heat-sensitive products or enzymes that may be heat-denatured. The water lost by evaporation has to be in many cases replenished, and this may lead to undesirable local increase in water activity during static processing.

Oxygen is also key factor affecting solid-state fermentation. Oxygen consumption rate and carbon dioxide production may be used to assess the state of the solid-state fermentation process, but different microorganisms cause these assessments to vary.

Important variations of 02 and CO2 concentration gradients seriously affect product yield. With gradient increases, the yield decreases. Forced ventilation may be used to suppress such gradient, but the addition of forced ventilation generates issues related to dehydration by evaporation and necessity of water addition that may result in undesirable local increase in water activity especially during static fermentation process.

Several bioreactor designs have been developed in an attempt to combat these problems, but only a few have been used at large-scale and they remain imperfect.

For example, perforated tray bioreactors have been developed. They are simple systems in which the substrate is laid out on trays which are perforated to facilitate air convection. For example, US6620614 discloses a solid-state fermenter for large scale fermentation, based on this technology. However, problems may also occur with oxygen transfer, which depends on tray characteristics and the height of the substrate layer. A uniform concentration of oxygen during the fermentation is difficult to obtain, because the formation of mycelium changes the porosity and thus the effective diffusivity of the states. The release of CO2 and heat also limits the transport of 02 and the creation of 02 gradients is inevitable. This results in an imperfect bioreactor or complex systems to limit the drawbacks of the bioreactor.

Another concept of bioreactors for Solid-State Fermentation was also developed, which is based on continuous or intermittent stirring of the solid cultured substrate. The bioreactors may be a rotating drum or horizontal paddle mixer with or without a water-jacket. Such bioreactors are expected to increase the homogeneity of the solid medium and to provide good oxygen transfer to the microorganism. They are however complex, and they may pose technical problems for large scale use concerning inoculation, sterilization of the substrate, harvest and cleaning in/of the bioreactor.

In the biogas field (CH4 gas produced through fermentation), many documents depict industry efforts to circumvent scale-up technical difficulties of larger and larger tanks or bioreactors. Many documents describe implementation of additional features, complex systems and sophisticated mechanisms to attain expected outputs: separators, double-chambers, three-section bioreactors, etc. While tanks or bioreactors of high volume (for instance tens of cubic meters) remain relatively simple, post fermentation add-ons are required to correct imperfections of the fermentation.

The present invention aims to address the above detailed problems of scaling up a Solid-State Fermentation.

According to the present invention, a process for large scale solid-state fermentation using microorganisms has been developed according to a counterintuitive approach, i.e. an approach contrary to developments known in the state of the art.

More particularly, the invention relates to a process for large scale solid-state fermentation. The process according to the invention comprises the steps of:
- providing a substrate to be cultured made of plant material and/or animal material;
- filling vessels with the substrate to be cultured, using an automated filling system, each vessel having three overall dimensions called height, width and depth measured in orthogonal directions, the smallest of the height, width and depth being less than or equal to 50 cm;

- sterilizing the filled vessels;
- inoculating the substrate, under aseptic conditions, with a microbial inoculant adapted to cause a solid-state fermentation of the substrate, the vessels being in a closed state after the inoculating step;
- storing the vessels in said closed state in controlled climate conditions during a time sufficient for fermentation of the substrate;
- harvesting the content of the vessel under food grade conditions.

The process may comprise a step of closing the vessel (S3) before the sterilizing state. In such case, the step of inoculating may comprise:
∘ opening the vessels,
∘ implanting the microbial inoculant on the substrate, and
∘ closing the vessels.

Contrary to the approach followed in the state of the art to scale-up a fermentation process and consisting in the development of large bioreactors, the present invention is based on the idea to perform Solid-State fermentation in small and simple reactors, but to increase the number of unitary reactors.

More particularly, using vessels whose smallest dimension is less than or equal to 50 cm promotes the gaseous homogeneity in the cultured substrates. For some applications, a smaller minimum dimension may be necessary, such as 40cm, 30cm or 20cm. Gaseous flows naturally happen in the vessel (e.g. by natural convection), and also between the inside volume of the vessel and the atmosphere through a filter, such as a porous membrane, preferably forming a microbial filter. Alcoholic fermentation is so avoided. No stirring of the cultured substrate is necessary, and no forced air circulation is necessary to keep the cultured substrate in an adapted state for Solid-State Fermentation. Thanks to the small size of the vessel, the heat generated by the fermentation is sufficiently transferred by conduction and convection to the walls of the vessels, and the surface area of said wall is sufficient to evacuate by convection enough heat into the air of the controlled climate area, so that the cultured substrate remains at an acceptably low and sufficiently homogeneous temperature.

The counterintuitive use of a large number of small vessels as Solid-State Fermentation reactors for large scale fermentation processes requires use of an automated process (and consequently automated means), in particular for filling the vessel, and optionally for the steps of closing the vessel, inoculating, harvesting, and for any steps of moving the vessels between the steps of the process. However, the automation of these steps has no drawback with regard to the fermentation itself.

Use of small size vessels for Solid State Fermentation at industrial scale has also provided additional advantages over the known process. Because each reactor or vessel has a small volume, each vessel used to produce a batch of fermented product may be used with a loading factor of approximately 100%. Furthermore, if any contamination of product happens, it will jeopardize only the content of a limited number of vessels. In addition, handling big volumes through numerous small units will also help to effectively achieve and maintain sterility. The process according to the invention is also highly flexible. The controls of moisture, temperature, airflow, etc. may be made in different rooms or areas for different batches or products. The process according to the invention thus makes it possible to run different fermentations in parallel using different substrates, microorganism and environmental conditions, but using the same equipment.

From an economic standpoint, this use of small size vessels to process a broader range of substrates allows modulation of operation to fit customers' needs. In particular, this makes it possible to handle substrates of disparate volumes, heterogeneous sources, and for different purposes. For instance, sources of substrates may vary depending on seasonality and weather conditions. Flexibility of the process is of tremendous importance for an industrial plant to optimize capital expenditure use throughout the year.

Harvesting under food grade conditions is conveniently performed by automated means, and makes it possible to produce food, pharmaceutical, biochemical products.

This automated process, intrinsically allowing great modularity and flexibility allows that same equipment be used for large scale industrial application in various application fields and sectors, where control and optimization of the fermentation, and homogeneity of outputs, are essential.

The microbial inoculant used in the process may comprise one or more bacteria, yeast, filamentous fungi, and microalgae. For example, fungi may be used belonging to one of the following fungal phyla (i.e. division): Basidiomycota and Ascomycota. In particular, the microbial inoculant may be a white-rot or brown-rot fungus, such as *Pleurotus sp,* or *Fomitopsis betulina* (formerly *Piptoporus betulinus).*

The substrate to be cultured may comprise fruit(s) or/and vegetable(s) or/and cereal(s) or/and pulse(s), and/or fruit(s) or/and vegetable(s) or/and cereal(s) or/and pulse(s) by-products, such as peel, fruit or/and vegetable pomace, and seeds, and/or cereal(s) or/and pulse(s) by-products.

For example, acorn squash, açai, alfalfa sprouts, allium (leeks, chives, garlic, garlic chives), amaranth, angelica, anise, anise hyssop, apple, apricot, artichoke, arugula, asian pear, asparagus, atemoya, avocado, azuki beans (or adzuki), bachelor's button, bamboo shoots, banana, banana squash, barley, basil, bean sprouts, beans, bee balm, beets, belgian endive, bell peppers, berries, bitter melon, black beans, black mustard, blackberries, black-eyed peas, blueberries, bok choy, boniato, borage blossoms, borlotti bean, boysenberries, broad beans, broccoflower, broccoli, brussels sprouts, buckwheat (smartweed family), butternut squash, cabbage, cactus pear, calendula/marigold, canary grass, cantaloupe, carambola, caraway, carnations/dianthus, carrots, casaba, cassava, melon, cauliflower, cayenne pepper, celery, chamomile, chamomile, chayote, cherimoya, cherries, chervil, chia, chickpeas, chicory, chili pepper, chives, chrysanthemum, cilantro, citrus (orange, lemon, lime, grapefruit, pomelo, mandarin, kumquat, etc.), clover flowers, coconuts, coix lacryma-jobi var. ma-yuen, collard greens, common beans, common peas (garden peas), coriander, corms, corn, courgette, cranberries, cucumber, daikon, dandelion, dates, delicate, dill, durum wheat, dried plums, eddoe, eggplant, endive, english daisy, escarole, fava beans, feijoa, fennel, fiddleheads, figs, finger millet, flax seed (flax family), fonio, foxtail millet, frisee, fuchsia, garlic, gem squash, ginger, gladiolus, gooseberries, grapefruit, grapes, green beans, green onions, greens, guava, habanero, hemp seed (hemp family), hibiscus, hollyhock, hominy, honeydew melon, horned melon, horseradish, hubbard squash, iceberg lettuce, impatiens flowers, india mustard, jalapeño, japanese millet, jasmine, jerusalem artichoke, jicama, Job's Tears, Johnny jump-up, kale, kañiwa, kidney beans, kiwicha, kiwifruit, kodo millet, kohlrabi, konjac, kumquat, lavender, leeks, lemon grass, lemon verbena, lentils, lettuces, lilac, lima beans, limes, longan, loquat, lupins, lychee, matcha, madarins, maize, Malanga, mangel-wurzel, mangetout or snap peas, marjoram, marrow mangos, millet, mint, mulberries, mung beans, mustard, mustard greens, napa, nasturtium, navy beans, nectarines, nettles, new zealand spinach, oats, okra, onion, oregano, papayas, paprika, parsley, parsnip, passion fruit, peaches, peanuts, pearl millet, pears, peas, peppers, persimmons, pigeon peas, pineapple, pinto beans, pitseed goosefoot, plantains, plums, pomegranate, poppy seed, potatoes, prickly pear, proso millet, prunes, pumpkin, quince, quinoa, radicchio, radish, radishes, raisins, rapeseed (including canola), raspberries, red cabbage, rhubarb, rice, romaine lettuce, rose, rosemary, runner beans, rutabaga, rye, safflower, sage, sage blossoms, salsify (oyster plant), scallion, shallots, skirret, snow peas, sorghum, soybeans, spaghetti squash, spelt, spinach, sprouts, squash, strawberries, string beans, sugarcane, sunflower, sweet potato, sweetcorn, tabasco pepper, tangelo, tangerines, taro, tatsoi, teff, tiger nut, thyme, tomatillo, tomato, topinambur, triticale, turnip, ugli fruit, valerianella, violets, wasabi, water chestnuts, watercress, watermelon, waxed beans, wheat, white radish, wild rice, yams, yellow squash, yuca/cassava, zucchini squash.

The substrate to be cultured may comprise (or be made of) products and/or by-products of the agro and/or food industry, such as fruit or vegetable pomace. By-product generally relates to a product that is produced as a result of making another product. Pomace is a typical by-product of fruit juice production; sugar beet pomace is a by-product of beet sugar production. Peels and seeds are also common by-products of the food industry.

The used by-products may depend on the production area and on the season, i.e. the period of the year. The process may use by-products having a low value, or by-products difficult to exploit by the agro-industry. For example, grape marc is widely available in the wine regions in autumn.

The vessels used in the process may be for example bottles or bags. Bags generally refers to a vessel with floppy walls, that is deformed when it is filled. The above indicated maximum smallest dimension of the vessel used in the process refers in this case to the dimension of a fully filled bag.

The vessels are advantageously made of food-grade material. For example, polyethylene or polypropylene (also known as polypropene or poly (1-methylethylene)) may be used. Stainless steel may be used.

Each of the vessels may have a maximum capacity comprised between 0.5L and 50L, and preferably between 1L and 10L. Use of small vessels is preferred for the above-mentioned reasons. This includes a possible use of vessels having a maximum capacity comprised between for example 0.5L or 1 L and 5L, 10L, 15L, 20L, 30L, 40L and 50L.

The process may be continuous or performed by batches. The process may mix continuous steps (i.e. steps performed continuously without planned interruptions) and steps performed by batches of vessels. The sterilizing step may be performed, by way of example, by batches of at least 100 vessels, preferably at least 500 vessels. The size of the batches used for the sterilizing step may depend on the size of a sterilization chamber where the sterilizing step is performed. Continuous sterilization hydrostatic (or equivalent horizontal technologies) retorts may also be considered.

After the harvesting step, a step of cleaning the vessels for re-use in an upcoming same process may be performed.

The invention also relates to a process for manufacturing a fermented flour comprising:
- performing the process for large scale solid-state fermentation as above described, and
- drying and grinding the fermented substrate to form the fermented flour.

The drying and grinding steps may be performed simultaneously or sequentially.

The invention also relates to a production system for performing a process for large scale solid-state fermentation as described above. The production system comprises:
- a substrate to be cultured supply hopper,
- an automatic conveying system,
- an automatic filling system, for filling the vessels,
- a sterilization system (such as a sterilization chamber for sterilization by batch, or a system for continuous sterilization),
- an automatic inoculating system
- a controlled climate area for stocking the filled vessels, and
- an automatic emptying system, for emptying the vessels under food grade conditions.

Such production system results in a possibly fully automated process, which can perform all steps needed in a fast and consistent way with the aim to produce fermented products at high throughput under Solid State Fermentation.

In this production system, each vessel in the closed state may comprise a filter such as a porous membrane allowing gaseous exchanges between an internal volume of the vessel and the controlled climate area.

The filter may in particular be carried by a cap used to close the vessel. The filter may be placed on other parts of the vessel. The closed vessel may comprise one or several filters or membranes. The filter or membrane allows gas exchange, but blocks contaminants such as spores, bacteria or other contaminants. For example, bacteria filtration is obtained with a membrane having a pore diameter of 40 micrometers or less.

The automatic conveying system of the production system may comprise a conveyor belt. This is a well-known and convenient automatic conveying system. Other automatic conveying systems may be used in the production system as a complement or alternative to conveyor belts. For example, automated guided vehicles may be used. The vehicle can implement various automation technologies for its guidance, which may be accomplished by wire guidance, optoguidance, and / or by more flexible technologies based on geolocation of the vehicle.

The invention also relates to a production system for manufacturing a fermented flour, wherein the system comprises, in addition to the above described system, a dryer and a grinding machine. The dryer and the grinding machine may, in some embodiments, be formed by a single machine such as a rotor pulverizer and dryer.

When the vessels used are bottles, the automatic filling system may comprise a bottler.

Other particularities and advantages of the invention will also emerge from the following description.

In the accompanying drawings, given by way of non-limiting examples:
- Figure 1 is a schematic block diagram representing a process according to an embodiment of the invention;
- Figure 2 schematically represents a production system according to an embodiment of the invention;
- Figure 3a and Figure 3b represent example embodiments of vessels that may be used in a process or in a production system according to the invention.

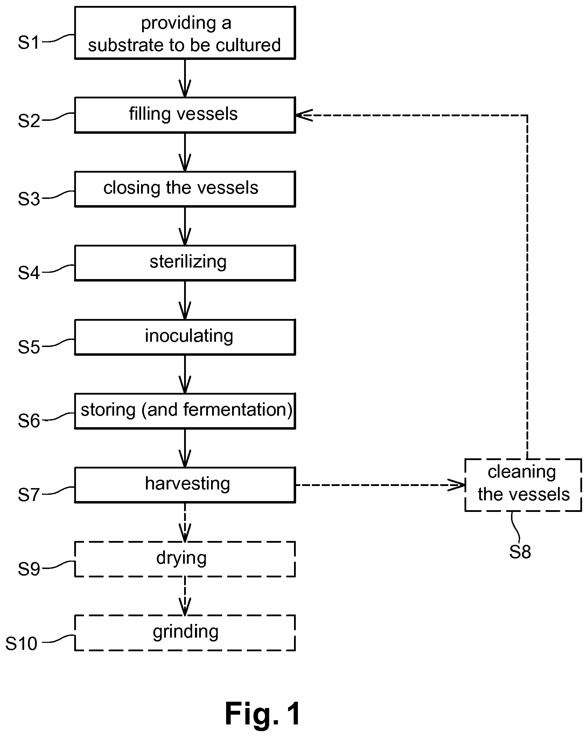

Figure 1 represents a process for manufacturing a fermented flour according to an embodiment of the invention. This process comprises a process for large scale solid-state fermentation using microorganisms according to a first aspect of the invention (which might generally aim at producing a fermented substrate, enzymes, biomass, organic acids and other metabolites), and additional steps to obtain a fermented flour.

The process of Figure 1 comprises a step providing a substrate to be cultured (S1). At this step, the substrate is prepared or provided. Preparing the substrate may comprise for example crushing, boiling, steaming, drying, hydrating, pressing, washing or mixing raw materials. The substrate, or fermentation medium, corresponds to the culture material where a microorganism will develop and cause a Solid State Fermentation.

The substrate to be cultured may comprise plant material (including fruit, vegetables, legumes, grains, pulses, flowers, leaves, grass, algae and their by-products), and/or animal material (including meat, fish, and dairy products and their by-products). These materials may be used alone or in combination (i.e. the substrate may be formed of a mix of these materials).

Vessels are then provided and filled with the substrate in a step of filling vessels S2 with the substrate to be cultured. While the vessels are generally almost completely filled with substrate, air can remain inside each filled vessel, and air is present in the mass of introduced substrate.

The vessels used as reactor in a process according to the invention are small sized vessel. Each vessel is preferably identical, having the same shape and same inside volume. More particularly, each vessel has at least one small dimension, i.e. a height, a width or a depth less than or equal to 50 cm, or less (such as 40cm, 30cm or 20cm). Height, width and depth are measured in orthogonal directions. For a vessel conformed to rest on a base, on a horizontal surface such as on the ground, height is measured in the vertical direction, and corresponds to the dimension from the base of the vessel to its top (i.e. its higher point). Width corresponds to the greatest dimension of the vessel that is orthogonal to its height. Depth is the third dimension of the vessel, depth being measured in a direction orthogonal to height and width.

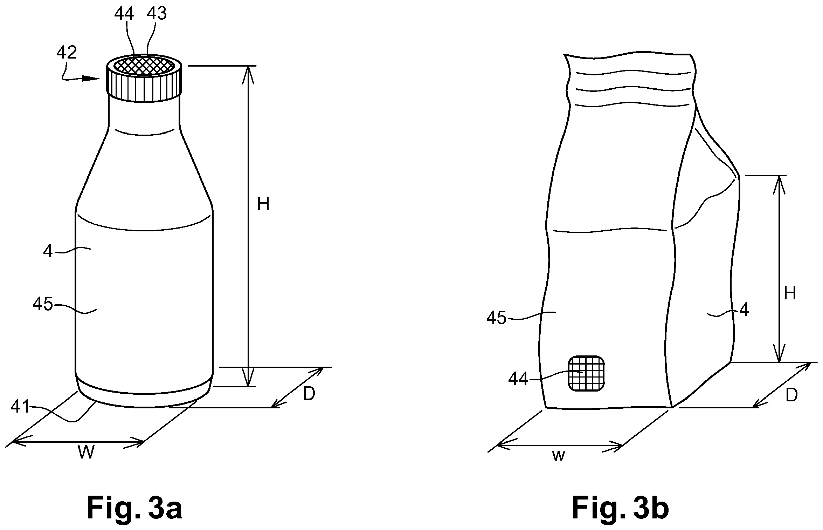

Examples of the height H, the width W and the depth D of different vessels are shown in Figure 3a and Figure 3b.

Because at least one of these three dimensions is small (e.g. less than 50cm), every point inside a vessel is situated at a short distance from a wall of the vessel (e.g. 25cm at most), so that heat transfer may occur from this point to the atmosphere through the wall of the vessel, and circulation of gas inside the vessel is not strongly impaired.

To fill relatively small reactors with a large quantity of substrate to be cultured, the step of filling vessels S2 with the substrate to be cultured is performed by an automated filling system, such as a bottler.

Optionally, the substrate may be optimized after having been filled into the vessels by perforation to form hole(s) in the substrate, or by shaking the filled vessel to accommodate the substrate but without pressing it.

In a step of closing the vessels S3, each filled vessel is closed.

After the closing step (S3), the vessels may be water-tightly and optionally air-tightly closed.

The closed vessels and their content are then sterilized in a sterilizing step S4. Sterilization may be performed by batches of vessels in a sterilization chamber.

The sterilization step is performed to inactivate microbes and their resistant structures (e.g.; spores). According to several embodiments of the invention, sterilization may be performed by using heat combined or not with high-pressure (e.g.; autoclaves, retorts, etc.) or by other alternative methods such as high temperature sterilization, low temperature sterilization, high-pressure sterilization, low-pressure sterilization, irradiation or chemical sterilization. The preferred type of sterilization for producing a fermented flour product to be used as a food product is a sterilization that can cause the destruction of all microorganisms in the substrate (whether or not pathogenic) and their spores, such as the heat combined with high-pressure sterilization.

After sterilization and if necessary cooling down, in an inoculating step (S5), the substrate contained in the vessels is inoculated with a microbial inoculant.

If the vessels have been air-tightly closed for sterilization, they may be stored a certain time before inoculation.

The inoculation comprises: opening the vessels, implanting the microbial inoculant such as spores, mycelia, cells, etc. on the substrate, and closing the vessels again. As for filling the vessels, this step comprising multiple but simple operations, which have to be carried out for each vessel is preferably performed by automated means. This step is performed in aseptic conditions, to avoid any contamination of the substrate during inoculation.

Alternatively, the vessels may be kept open for sterilization and inoculation, and be closed only after inoculation.

After the inoculating step, the vessels are in a closed state. For example, a cap is placed on a neck of the vessel, or another closing system such as a sliding mechanism for seal a bag, or other sealing mechanism.

The vessels, in the closed state, are water-tightly sealed. However, gas exchanges with the atmosphere surrounding the vessel must be possible during fermentation. For that purpose, each vessel has one or several filters (e.g. at the level of the cap used to close the vessel after implantation of the microbial inoculant) that allow gas exchanges, but block contaminants such as spores, bacteria or other contaminants. A porous membrane may be used, more particularly a bacteria filtering hydrophobic porous membrane.

As previously indicated, the microbial inoculant may be one or more bacteria, yeast, filamentous fungi, and microalgae. Selection of the microorganism used in the microbial inoculant depends among other parameters on the final product sought, on its ability to develop in one or many type(s) of given substrate, on its availability and price, etc.

Thus, the microbial inoculant used in the process may comprise one or more of bacteria (including actinomycetes), yeasts, and filamentous fungi; it being genetically modified or not. For example, one or several microorganisms of the below listed genera can be used in the invention.

### Bacteria & Actinomycetes

*Acetobacter, Actinomyces, Acinetocacter, Actinoplanes, Actinomadura, Aerococcus, Aeromonas, Alcaligenes, Alcanivorax, Alloiococcus, Alteromonas, Amycolatopsis, Anabaena, Arthrobacter, Arthrospira, Atopobium, Bacillus, Bifidobacterium, Brevibacterium, Brevundimonas, Carnobacterium, Catenisphaera, Cellulomonas, Citrobacter, Clostridium, Corynebacterium, Cyanobacteria, Dermatophilus, Desulfotomaculum, Dietzia, Enterobacter, Enterococcus, Escherichia, Frankia, Flavobacterium, Geobacillus, Gluconobacter, Gordonia, Humicola, Janthinobacterium, Lactobacillus, Lactococcus, Leuconostoc, Klebsiella, Marinobacter, Microbacterium, Micromonospora, Microtetraspora, Moraxella, Mycobacterium, Mycococcus, Micrococcus, Nocardia, Oenococcus, Pediococcus, Phormidium, Propionibacterium, Pseudomonas, Raoultella, Rastonia, Rhizobium, Rhodococcus, Saccharopolyspora, Serratia, Shigella, Sphingomonas, Staphylococcus, Streptococcus, Streptomyces, Symbiobacterium, Synechococcus, Synechocystis, Tetragenococcus, Thermoactinomyces, Thermomonospora, Vagococcuswhich, Vibrio, Weissella, Xanthomonas.*

### Yeasts

*Achromobacter, Arxula, Aureobasidum, Blastobotrys, Brettanomyces (its perfect stage, Dekkera), Candida, Citeromyces, Cryptococcus, Cystofilobasidium, Debaryomyces, Endomycopsis, Filobasidiella, Galactomyces, Geotrichum, Glaciozyma, Guehomyces, Hansenula, Hanseniaspora (its asexual counterpart Kloeckera), Hyphopichia, Kluyveromyces, Kodamaea, Komagataella, Lachancea, Lipomyces, Metschnikowia, Moniella, Mrakia, Ogataea, Pichia, Phaffia, Pseudozyma, Rhodotorula, Rhodosporidium, Starmerella, Saccharomyces, Saccharomycodes, Saccharomycopsis, Scheffersomyces, Schizosaccharomyces, Schwanniomyces, Torulopsis, Torulaspora, Trichosporon, Trigonopsis, Yarrowia, Xanthophyllomyces and Zygosaccharomyces*

### Filamentous fungi

*Acremonium, Agaricus, Agrocybe, Akanthomyces, Alternaria, Ampelomyces, Amylosporus, Antrodia, Armillaria, Ashbya, Aspergillus, Atkinsonella, Aureobasidium, Auricularia, Balansia, Balansiopsis, Beauveria, Bispora, Bjerkandera, Boletus, Cantharellus, Catenaria, Cephalosporium, Chaetomium, Chrysonilia, Cladosporium, Claviceps, Clitocybe, Clitopilus, Colletotrichum, Collybia, Coniochaeta, Coprinus, Cordyceps, Coriolus, Cunninghamella, Cyathus, Cyclocybe, Cylindrocarpon, Cylinrocarpum, Cytonaema, Cytospora, Daldinia, Dentipellis, Doratomyces, Echinodothis, Emericella, Emericellopsis, Entoloma, Epichloe, Epicoccum, Favolaschia, Flammulina, Fomes, Fomitopsis, Fusarium, Ganoderma, Giberella, Gliocladium, Grifola, Gymnoascus, Hericium, Hohenbuehelia, Hormonema, Humicola, Hydropus, Hypomontagnella, Hypomyces, Hypoxylon, Hypsizigus, Inocutis, Inocybe, Inonotus, Isaria, Kuehneromyces, Lactarius, Laetiporus, Laxitextum, Lecanicillium, Lentinula, Lentinus, Lepista, Leptoshaeria, Lignosus, Lycoperdon, Lyophyllum, Martierella, Metarhizium, Monascus, Monilia, Monocillium, Morchella, Mortierella, Mucor, Mycelia, Myriogenospora, Neurospora, Nigrospora, Omphalotus, Ophiocordyceps, Oudemansiella, Paecilomyces, Panellus, Panus, Paraconiothyrium, Paraepichloë, Penicillium, Peniophora, Periconia, Pestalotiopsis, Phellinus, Phlebia, Pholiota, Phoma, Phomopsis, Piptoporus, Pleurotus, Pochonia, Polyporus, Preussia, Pycnoporus, Ramaria, Rhizopus, Rhodotus, Sarcodon, Schizophyllum, Scytalidium, Scytalidium, Scytinostroma, Sparassis, Spicaria, Stachybotrys, Steccherinum, Stropharia, Suillus, Thermoascus, Thermomyces, Tolypocladium, Torula, Trametes, Tremella, Trichoderma, Tricholoma, Tuber, Verticillium, Volvariella, Wolfiporia, Wrightoporia, Xylaria.*

Each closed vessel is then placed, in a closed state, in a controlled climate area, for a storing step (S6). The temperature in this area is controlled to promote the development of the microorganism in the cultured substrate and fermentation. A temperature of 10°C to 50°C is generally appropriate (depending on the substrate and on the microbial inoculant). Other controlled parameters in the controlled climate area may be hygrometry, oxygen and CO₂ levels.

Solid State Fermentation happens during the storing step. This step is carried on until colonization of the cultured substrate is complete (or almost complete). By way of example, the storing step may last from 3 to 60 days, depending on the volume of the vessel, the climate conditions, the cultured substrate and the microbial inoculant.

After colonization, the fermentation vessel is emptied and the fermented product is collected (harvesting step S7). Harvesting is generally performed under food grade conditions, to avoid contamination of the fermented product. Food grade generally refers to materials being non-toxic and safe for consumption, or, more simply said, being safe for food.

A fermented product is so obtained, in large quantity, thanks to a process using multiple simple fermentation vessels instead of a large and complex bioreactor.

Advantageously, the emptied vessels are cleaned for reuse (in a step of cleaning the vessels S8), i.e. to be sent to the step of filling vessels S2 with substrate of a further same process.

The above described process example for solid-state fermentation may be follow by a step of drying S9 the fermented product. The fermented dried product may be ground (step of grinding S10) to form a flour. The steps of drying and grinding may be performed simultaneously.

The obtained fermented flour may be of high nutritional interest. It has generally a low sugar content, and has a high fiber content. It may be gluten free.

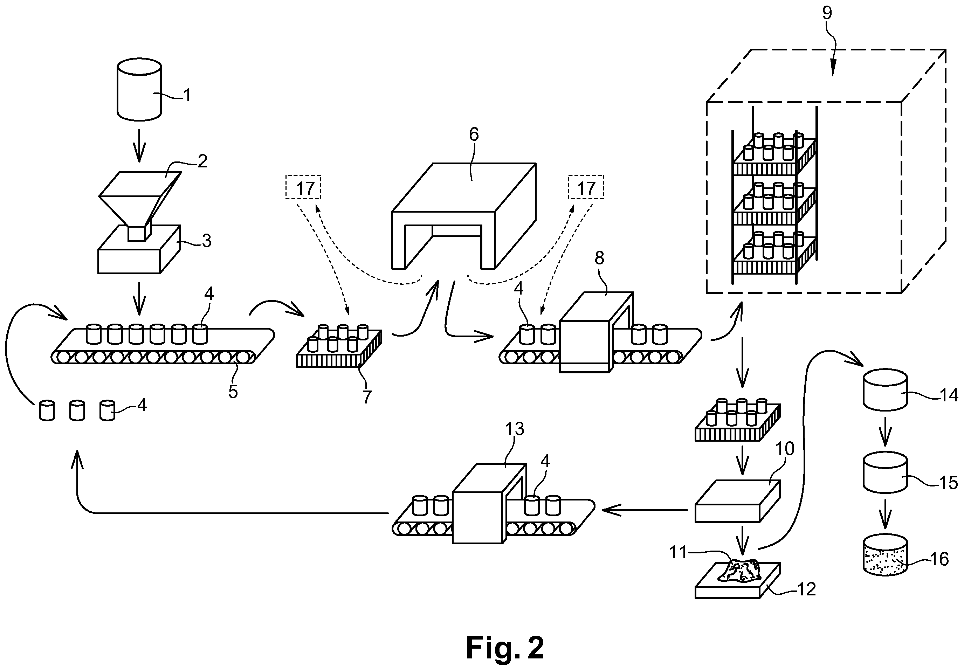

Figure 2 schematically represents a production system according to an embodiment of the invention.

The production system corresponds to a set of machines and equipment, installed in one or several buildings, used to performed a process for large scale solid-state fermentation according to the invention.

The production system may comprise one or several items of equipment for substrate preparation 1, such as a crusher, a boiler, a steamer, a washer, a presser, and/or a mixer.

The system also comprises a hopper 2, or a similar item, for collection of the prepared (or directly provided) substrate to be cultured. The substrate to be cultured is distributed from the hopper to an automatic filling system 3. The automatic filling system 3 is adapted to fill vessels 4 with a predefined quantity (volume or mass) of substrate. The automatic filling system 3 may also adapted to close, for example to cap, the filled vessels 4. If the vessels 4 are bottles, the automatic filling system 3 may comprise or may be a bottler.

The automatic filling system may be formed of two distinct machines respectively for filling the vessels and for closing the vessels.

The vessels are provided to the automatic filling system 3 by an automatic conveying system 5 such as a conveyor belt.

The filled vessels are then sent to a sterilization chamber 6, for sterilization of the vessels and of their content. For this operation, in particular if a sterilization chamber is used, the filled and closed vessels may be grouped in batches, e.g. they may be placed on pallets 7 or suitable crates to be manually or automatically transported into the sterilization chamber 6. Manual transport corresponds to a transport using for example a human guided manual or electric pallet truck. Automatic transport refers to a transport by an automatic conveying system, such as a conveyor belt, a lift system or an automatic guided vehicle.

While the represented production system comprises a sterilization chamber, other systems performing a continuous sterilization such as continuous sterilization retorts may be used. In such case, the vessels may be placed, after sterilization, on pallets 7 or suitable crates to be manually or automatically transported.

After sterilization, the (sterilized) substrate to be cultured is inoculated with a microbial inoculant. This step, comprising at least the operations of opening each vessel, implanting the microbial inoculant(s) on the substrate, and closing the vessels, is preferably performed in an automatic inoculating system 8. The vessels are preferably transported (e.g. de-palletized and carried) to the automatic inoculating system 8 by an automatic conveying system. Opening, implanting the microbial inoculant and closing the bottle is performed in aseptic conditions inside the automatic inoculating system 8.

To optimize the production process, one or several buffers 17 may be provided, where vessels can be stored between two steps of the process. For example, filed vessels may be stored before the sterilization step. This may improve operation efficiency and be especially useful in the year round treatment of seasonal supply raw materials/substrates

After the sterilization step, provided that the vessels are closed, the vessels may be stored before the inoculating step. It should be noted that in such case, the caps (for example) used to close the vessels for sterilization and further storing may be different (e.g. provide an airtight closure) from the caps used to close the vessels after inoculation (to allow gas exchanges).

The production system further comprises a controlled climate area 9. The controlled climate area is an area which is adapted to storing the vessels, e.g. in grouped and/or palletized form for example in racks or shelves, and where the environmental parameters are controlled.

The controlled parameters may comprise temperature, hygrometry, oxygen level and carbon dioxide level, and light or darkness. Sensors may be installed in the controlled climate area 9 to measure one or several environmental parameters, and corrections means may be used if any parameter drifts outside a predefined value range. The controlled climate area may be provided with a heater and/or an air conditioner, an air desiccator or a fogger, a ventilation system, etc.

Of course, several controlled climate areas may be arranged in the production system. This makes it possible to carry out several fermentation processes in parallel, with, if needed, different environmental parameters.

An automatic emptying system 10 is provided, for emptying the vessels of their content after fermentation.

The automatic emptying system allows harvesting of the fermented product under at least food grade conditions, for fermented food products. For other products, such as a pharmaceutical product or a biochemistry product, harvesting may be performed under sterile or aseptic conditions.

After harvesting, a large quantity of bulk fermented product 11 is obtained, for example in a fermented product receptor 12.

The emptied, used, vessels may be then cleaned, for example in a vessel cleaning machine such as a cleaning tunnel 13. The cleaned vessels may then be reused.

The represented production system is a system for producing a fermented flour. The bulk fermented product is provided to a dryer 14, where it is dried. The dried bulk fermented product is ground in a grinding machine 15.

A fermented flour 16 is obtained and packed.

Figure 3a and Figure 3b represent example embodiments of vessels that may be used in a process or in a production system according to the invention.

Figure 3a shows a vessel having the form of a bottle. The bottle has a flat base 41 adapted to rest on a flat surface. Opposite to the base 41, the bottle has an open neck 42 that may be used to fill and empty the bottle. The size of the neck must thus be sufficient in particular to collect the cultured substrate after fermentation.

The height H of the bottle is defined as the dimension from the base 41 to the top of the neck 42. The represented bottle has a cylindrical general shape. The width W and the depth D of the bottle are thus the same, and are equal to the diameter of the bottle. The height H is, in the represented example, the greatest dimension of the bottle, and the width W and the depth D are the smallest dimensions of the bottle. They do not exceed 50 cm.

The bottle could of course have a different general shape. The smallest dimension could be its height. It could have any other prismatic general shape.

The bottle of Figure 3a comprises a cap 43. The cap 43 is provided with a filter 44, for example a microbial filtering porous membrane. The cap 43 may close the neck of the bottle and be attached to it according to several alternative known arrangements: it may for example be a screw cap, or it may be fitted to the neck by snap engagement. The interface between the cap and the neck is airtight, but the filter 44 allows gas exchanges between the inside and the outside of the vessel 4

The walls 45 of the bottle may be formed in various food grade materials. It may be made of plastics, such as polyethylene (PE), polypropylene (PP), silicone, glass or stainless steel. Because the walls have to allow heat exchanges between the cultured substrate contained in the bottle and the atmosphere around the bottle, thin plastic walls or thin stainless steel walls are preferred.

Figure 3b shows another embodiment of a vessel that may be used in a process or in a production system according to the invention. In the embodiment of Figure 3b, the vessel is formed of a bag. The vessel basically comprises a thin-walled pouch made of a deformable plastic material. In the represented embodiment, once filled, the smallest dimension of the height (H); width (W) and depth (D) of the bag does not exceed 50 cm.

The bag comprises an opening 46 that is sealed (as shown in Figure 3b) after inoculation of the substrate to be cultured comprised in the bag. For example, the bag comprises a top part that is folded for closing the bag before sterilization After sterilization, thermosealing may be used to seal the bag. Other known closing systems may be used, such as a sliding mechanism, or the bag may be provided with port that can be closed by an adapted cap. A large opening facilitates emptying of the vessel.

In the represented embodiment, the wall 45 of the bag is provided with a filter 44 which allows gas exchanges between the inside and the outside of the vessel. In embodiments wherein the bag is closed by a cap, another filter may be provided on the cap 43 in alternative or complement to the filter provided on the wall of the bag.

The process and the system developed in the invention makes it possible to produce a fermented product at a large scale, i.e. in large quantities, without the drawbacks of the systems known in the state of the art. This result is obtained by an unknown and counterintuitive approach in the field of bioprocessing, this approach consisting in upscaling a process by providing a high number of small and simple bioreactors and by using a high level of automation instead of increasing the size of the reactors.

The invention results in a highly automated and highly flexible process, which has many advantages over the known processes, in terms of safety, environmental friendliness, ability to use by-products of the food industry and to accommodate seasonal variations of the available by-products, etc.

The invention is particularly adapted to the production of a fermented flour for the food industry.

## Claims

1. Process for large scale solid-state fermentation, the process comprising the steps of:
• providing a substrate to be cultured (S1) made of plant material and/or animal material;
• filling vessels (S2) with the substrate to be cultured, using an automated filling system (3), each vessel (4) having three overall dimensions called height (H), width (W) and depth (D) measured in orthogonal directions, the smallest of the height (H), width (W) and depth (D) being less than or equal to 50 cm;
• sterilizing (S4) the filled vessels;
• inoculating (S5) the substrate, under aseptic conditions, with a microbial inoculant adapted to cause a solid-state fermentation of the substrate, the vessels being in a closed state after the inoculating step;
• storing (S6) the vessels in said closed state in controlled climate conditions during a time sufficient for fermentation of the substrate;
• harvesting (S7) the content of the vessels under food grade conditions.

2. Process according to Claim 1, wherein it comprises a step of closing the vessels (S3) before the sterilizing state, and wherein the inoculating (S5) step comprises:
∘ opening the vessels,
∘ implanting the microbial inoculant on the substrate, and
∘ closing the vessels.

3. Process according to Claim 1 or Claim 2, wherein the microbial inoculant comprises one or more bacteria, yeasts, filamentous fungi, and/or microalgae.

4. Process according to Claim 3, wherein the microbial inoculant comprises fungi belonging to one of the following fungal phyla: Basidiomycota and Ascomycota.

5. Process according to Claim 4, wherein the microbial inoculant is a white-rot or brown-rot fungus, and preferably one of *Pleurotus sp and* Fomitopsis betulina (formerly *Piptoporus betulinus*).

6. Process according to any one of the preceding Claims, wherein the substrate to be cultured comprises fruit(s) or/and vegetable(s) or/and cereal(s) or/and pulse(s), and/or fruit(s) or/and vegetable(s) or/and cereal(s) or/and pulse(s) by-products, such as peel, fruit or/and vegetable pomace, and seeds, and/or cereal(s) or/and pulse(s) by-products.

7. Process according to any one of the preceding Claims, wherein the substrate to be cultured comprises product(s) and/or by-product(s) of the agro and/or food industry.

8. Process according to any one of the preceding Claims, wherein the vessels (4) are bottles or bags.

9. Process according to Claim 8, wherein the vessels are made of food-grade material, and preferably made of polyethylene (PE), polypropylene (PP) or stainless steel.

10. Process according to any one of the preceding claims, wherein each of the vessels has a maximum capacity comprised between 0.5L and 50L, and preferably between 1L and 10L.

11. Process according to any one of the preceding Claims, the step of sterilizing being performed by batches of at least 100 vessels, preferably at least 500 vessels.

12. Process according to any one of the preceding Claims comprising, after the harvesting step, a step of cleaning the vessels (S8) for re-use in an upcoming same process.

13. Process for manufacturing a fermented flour comprising:
• performing the process for large scale solid-state fermentation of one of claims 1 to 12; and
• drying (S9) and grinding (S10) and/or milling the harvested content of the vessels to form the fermented flour.

14. A production system for performing the process for large scale solid-state fermentation of one of claims 1 to 12 comprising:
• a substrate to be cultured supply hopper (2);
• an automatic conveying system (5);
• an automatic filling system (3), for filling the vessels (4);
• a sterilization system (6);
• an automatic inoculating system (8);
• a controlled climate area (9) for stocking the filled vessels, and
• an automatic emptying system (10), for emptying the vessels under food grade conditions.

15. A production system according to Claim 14, wherein each vessel (4) in the closed state comprises a filter (44) such as a porous membrane allowing gaseous exchanges between an internal volume of the vessel (4) and the controlled climate area (9).

16. A production system according to Claim 14 or Claim 15, wherein the vessels are bottles and the automatic filling system comprises a bottler.
